(19) Europäisches Patentamt
European Patent Office
Office européen des brevets

(11) **EP 2 039 350 A1**

(12) **EUROPEAN PATENT APPLICATION**
published in accordance with Art. 153(4) EPC

(43) Date of publication:
**25.03.2009 Bulletin 2009/13**

(21) Application number: **07768061.9**

(22) Date of filing: **03.07.2007**

(51) Int Cl.:
*A61K 9/127* (2006.01)  *A61K 47/24* (2006.01)
*A61K 47/28* (2006.01)  *A61K 47/34* (2006.01)
*B01D 15/04* (2006.01)  *G01N 30/02* (2006.01)
*A61K 31/704* (2006.01)  *A61P 35/00* (2006.01)

(86) International application number:
**PCT/JP2007/063287**

(87) International publication number:
**WO 2008/004542 (10.01.2008 Gazette 2008/02)**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(30) Priority: **03.07.2006 JP 2006183282**

(71) Applicant: **Terumo Kabushiki Kaisha
Tokyo 151-0072 (JP)**

(72) Inventor: **YOSHINO, Keisuke
Ashigarakami-gun
Kanagawa 259-0151 (JP)**

(74) Representative: **TBK-Patent
Bavariaring 4-6
80336 München (DE)**

(54) **METHOD OF SEPARATING VESICLE, PROCESS FOR PRODUCING MEDICINAL PREPARATION, AND METHOD OF EVALUATION**

(57) A method of separating vesicles whose membrane has been modified with a hydrophilic polymer (polyethylene glycol, etc.). In the method, vesicles (liposome, etc.) differing in the degree of membrane modification are separated by ion-exchange chromatography using a concentration gradient in which the ionic intensity of an eluent is changed with time. Also provided are: a process for producing vesicles purified so as to have a desired degree of modification with a hydrophilic polymer which comprises utilizing the separation method in the step of purifying vesicles; a method of evaluating a medicinal vesicle preparation containing a drug therein, the method comprising using the separation method; and vesicles in which the membrane has been modified with a hydrophilic polymer and which include no vesicle having a degree of membrane modification less than 0.5 mol%.

EP 2 039 350 A1

**Description**

Technical Field

This invention relates to a method for separating membrane-modified closed vesicles, a process for preparing a purified preparation by use of the separation method, and a method for evaluating a closed vesicle and preparation having a controlled, desired modification rate with a hydrophilic polymer.

Background Art

In recent years, extensive studies have been made on drug delivery systems (DDS) wherein a drug is safely and efficiently delivered to and distributed at the target lesion site. For one such approach, studies have been made on the use, as a transporter (carrier) of a drug, of a closed vesicle such as liposome, emulsion, lipid microsphere, nanoparticle or the like. For the practical use of DDS using such a closed vesicle, however, there are many problems to solve. Especially, in order to deliver a drug to the target site, it is important to allow the closed vesicle to escape from the foreign body recognition mechanism of a biological body and control disposition, e.g. to prevent aggregation through interaction (adsorption) with opsonin protein or serum protein in the blood and to avoid capturing in the reticuloendothelial systems (RES) such as liver, spleen and the like. That is, the stability in blood (retentivity in blood) of the closed vesicle is important.

The retentivity in blood is a property of causing a drug to exist in blood, and more improved retentivity in blood enables a drug to be administered in lesser amounts. It is considered that if the retentivity in blood of closed vesicles is enhanced, the vesicles can be passively accumulated at tissues with increased vascular permeability, for example, in the tumor tissue or in the inflammatory site, thereby enabling them to reach the target site at high selectivity.

One known method for solving the above problem is a technique of modifying the membrane forming the closed vesicle with a hydrophilic polymer. The closed vesicle modified with a hydrophilic polymer on the surface thereof can prevent opsonin protein in serum from being adsorbed on the surface to enhance stability in blood and can avoid capturing with RES. In particular, with the liposome formed of a phospholipid membrane, it has been confirmed that the retentivity in blood is improved by membrane modification with a hydrophilic polymer, thereby moving toward practical use of a liposome preparation of such a form (see Patent Documents 1 to 3 and Non-Patent Documents 1 to 3). The liposome, which has been subjected to membrane modification with polyethylene glycol (hereinafter referred to as "PEG") used as a hydrophilic polymer because it is low in toxicity and thus, has been widely applied so as to stabilize drugs and improve disposition, has been used to realize preparations of a variety of drugs.

In such membrane modification techniques of closed vesicle with a hydrophilic polymer as set out above, the modification amount of a hydrophilic polymer and the state of distribution thereof on the membrane surface become important factors relating to the functions of a preparation, i.e. the retentivity in blood and physicochemical stability of the preparation. Especially, even with the case of a hydrophilic polymer whose toxicity has been confirmed as being low, it is favorable to use a smaller amount of the polymer when taking into account administration into the living body. In addition, the membrane modification in excess is liable to cause instabilization of the membrane and is not favorable from the standpoint of production costs ascribed to preparation failure rather than from the standpoint of an expected retentivity in blood. From this view, modification of a preparation with a hydrophilic polymer should favorably be performed in a minimum requirement.

Hitherto, such modification with a hydrophilic polymer is usually discussed based on charge amount. In the membrane modification treatment of closed vesicle, an introduced amount of a hydrophilic polymer is generally calculated as a difference obtained by subtracting a quantitatively determined amount of the polymer not removed by a removal treatment after the modification treatment from a charge amount thereof. A method of directly analyzing an actually introduced water-soluble hydrophilic polymer material incorporated in the micelles has been proposed, i.e. a method has been proposed wherein the membrane (micelles) per se is solubilized with an aqueous solvent containing a surface active agent, followed by analysis with gel filtration chromatography (see Patent Document 4).

The physicochemical analyses of the surface state of nanosize particles have been hitherto made by measurements of a particle size and a zeta potential. Moreover, it has been reported that the state of PEG molecules on the surface of the liposome membrane is estimated from the fixed aqueous layer thickness (FALT) and the aqueous two-phase system, both formed on the surface of the liposome membrane, measured by interfacial-electrochemical technique, from which reference is made to the interrelation between the PEG modification and the increased retentivity in blood (see Non-Patent Documents 5 to 7).

Patent Document 1: JP-T-Hei 5-505173
Patent Document 2: Japanese Patent Publication No. Hei 7-20857

Patent Document 3: Japanese Patent No. 2667051
Patent Document 4: Japanese Patent Laid-Open No. Hei 10-142214
Non-Patent Document 1: "LIPOSOMES from Physics to Applications," written by D.D. Lasic, Elsevier, 1993 Non-Patent Document 2: "Long Circulating Liposomes: Old Drugs, New Therapeutics," edited by Martin C. Woodle and Gerrit Storm, Springer, 1997
Non-Patent Document 3: "Medical Applcations of LIPOSOMES," edited by D. D. Lasic and D. Papahadjopoulos, Elsevier, 1998
Non-Patent Document 4: "Effects of mixed polyethylene glycol modification on fixed aqueous layer thickness and antitumor activity of Doxorubicin containing liposome," edited by Yasuyuki Sadzuka, 238, 2002, 171 to 180
Non-Patent Document 5: "Liposome Technology 2nd Edition Volume I," edited by Gregory Gregoriadis, 1992
Non-Patent Document 6: "Liposomes in Life Science," edited by Hiroshi Terada, Springer-Verlag Tokyo, 1992
Non-Patent Document 7: Colin Tilcock, Pieter Cullis, Tomas Dempsey, B.B.A 979(1989), 208 to 214

Disclosure of Invention

Technical Problem

Desired membrane modification of a closed vesicle on the outer surface thereof as set out above is modification with a required minimum amount of a hydrophilic polymer, and especially, uniform membrane modification in a required minimum amount only on the outer surface of the closed vesicle is desirable. More particularly, if a variation throughout preparation lots is small and an average value analyzed as a content of a hydrophilic polymer is regarded substantially as a content of individual closed vesicle particles, it can be expected that the effect brought about by the hydrophilic polymer can be most efficiently developed. In particular, if a variation is great with respect to the average value, there is the high possibility that there exist either closed vesicle particles wherein the hydrophilic polymer is not contained in an expected amount, or conversely, closed vesicle particles wherein the polymer is contained in an excess amount that is inappropriate from the medicinal standpoint even if a desired content is attained as viewed in terms of a total average. Accordingly, there is demanded a method wherein the state of modification with a hydrophilic polymer can be analyzed with respect to individual closed vesicle particles. Using the method, it is desirable to obtain an actually effective rate of modification with a hydrophilic polymer. However, the modification amounts of the hydrophilic polymer measured according to the abovestated conventional methods are all obtained as an average of all samples being measured. According to these methods, no modification amount based on individual closed vesicles in a sample being measured can be obtained.

Technical Solution

Under these circumstances, we made studies so as to establish a method of analyzing a difference in the modification state with a hydrophilic polymer on an outer surface of a closed vesicle in order to obtain closed vesicles having a controlled, desired rate of modification with the hydrophilic polymer, whereupon we have obtained knowledge that closed vesicles can be separated depending on the rate of modification with the hydrophilic polymer by application, to ion-exchange chromatography, of a concentration gradient wherein the ionic intensity of an eluent is changed with time. This closed vesicle may be a preparation encapsulating a drug therein. It has been found that when this separation method is applied to a manufacturing process, there can be obtained closed vesicles (preparations) of high uniformity at a desired rate of modification with the hydrophilic polymer. For instance, the above separation method is directly carried out as a purifying measure of prepared closed vesicles (preparations), or the above separation method is applied as an analyzing method of closed vesicles (preparations), after which the resulting analyzed datas are fed back to a manufacturing process so as to control manufacturing conditions. By this, there can be prepared closed vesicles (preparation) whose distribution is narrow and which have high uniformity of a desired rate of modification with hydrophilic polymer.

Further, when the chromatogram obtained by the separation method and separately obtained effects of retentivity in blood for preparations of the respective rates of modification with a hydrophilic polymer are compared with each other, an exact interrelation between the rate of modification with the hydrophilic polymer of the preparations and the effects can be known.

With respect to the closed vesicles modified with a hydrophilic polymer, no method of directly analyzing the state of modification with the hydrophilic polymer has been known. Especially, it has never been reported that closed vesicles are separated as they are, or they are non-destructively separated depending on the difference in the state of an outer surface of the membrane with the respective hydrophilic polymers, or that the distribution thereof is known. Accordingly, the following inventions are provided so as to solve the above problems.

(1) A method for separating closed vesicles membrane-modified with a hydrophilic polymer, the method including separating closed vesicles differing in rate of membrane modification by use of ion-exchange chromatography applied with a concentration gradient wherein an ionic intensity of an eluent is changed with time.

(2) The separation method of (1) above, wherein the hydrophilic polymer is made of polyethylene glycol and the closed vesicle is made of a liposome.

(3) The separation method of (1) or (2) above, wherein the ionic intensity is an ionic intensity of NaCl.

(4) The separation method of any one of (1) to (3) above, wherein the ion-exchange chromatography is a weak anion-exchange chromatography.

(5) The separation method of (4) above, wherein the eluent is an aqueous solvent containing a buffer agent and having a pH of 6 to 10.

(6) The separation method of any one of (1) to (5) above, wherein the closed vesicle is a preparation containing a drug in the closed vesicle.

(7) A method for preparing closed vesicles, the method including:

providing closed vesicles subjected to membrane modification with a hydrophilic polymer;
separating the closed vesicles depending on the rate of membrane modification with the hydrophilic polymer by the separation method of the closed vesicles defined in any one of claims 1 to 6; and
collecting closed vesicles having a desired rate of modification with the hydrophilic polymer to obtain purified closed vesicles.

(8) The preparation method as recited in (7), wherein the membrane-modified closed vesicle is a liposome whose membrane is modified with polyethylene glycol.

(9) Closed vesicles modified with a hydrophilic polymer on an outer surface of a membrane thereof, which are free of closed vesicles wherein a rate of modification with the hydrophilic polymer is less than 0.5 mol% relative to a membrane lipid of the closed vesicle.

(10) The closed vesicles as recited in (9) above, wherein the hydrophilic polymer is made of polyethylene glycol and the closed vesicle is made of a liposome.

(11) The closed vesicles as recited in (9) or (10) above, wherein the closed vesicle is a preparation containing a drug therein.

(12) A method for evaluating a preparation of a closed vesicle membrane-modified with a hydrophilic polymer and containing a drug therein on the basis of a rate of modification with the hydrophilic polymer by use of the separation method recited in any one of (1)-(6) above.

(13) A method for preparing closed vesicles including the steps of preparing closed vesicles subjected to membrane modification with a hydrophilic polymer and analyzing the thus prepared closed vesicles according to the separation method of any one of claims 1 to 6, wherein preparing conditions in the preparing step are controlled in such a way that a predetermined rate of membrane modification with the hydrophilic polymer is obtained based on the resulting analyzed data thereby obtaining closed vesicles wherein the rate of membrane modification with the hydrophilic polymer is controlled at the predetermined rate of membrane modification.

Advantageous Effect

According to the invention, closed vesicles (intended to include preparations) having different membrane surface states owing to modification with a hydrophilic polymer can be separated from one another. A method for preparing closed vesicles having a controlled, desired rate of modification with a hydrophilic polymer can be provided wherein the separation method is utilized for a purifying step of closed vesicles. According to this preparation method, there can be obtained closed vesicles wherein individual closed vesicles mutually have a highly uniform rate of membrane modification. Especially, those vesicles wherein membrane modification with a hydrophilic polymer is at a level less than an effective rate of modification from the internal dynamic standpoint are removed and thus, there can be provided only closed vesicles purified as having an effective rate of membrane modification.

In the practice of the invention, a precise rate (with a narrow distribution) of surface modification can be known, for which the relation between the rate of surface modification and the preparation effect can be exactly evaluated.

Brief Description of Drawings

[Fig. 1] (A) to (C) are a view showing measuring conditions in the method of the invention, respectively.

[Fig. 2] Fig. 2 is a high-performance liquid chromatogram indicating an analytic example (separation state) of a PEG-modified liposome preparation using conditions 1 in the method of the invention.

[Fig. 3] Fig. 3 is a high-performance liquid chromatogram indicating an analytic example (separation state) of a PEG-modified liposome preparation using conditions 2 in the method of the invention.

[Fig. 4] Fig. 4 is a high-performance liquid chromatogram indicating an analytic example (separation state) of a PEG-modified liposome preparation using conditions 3 in the method of the invention.

[Fig. 5] Fig. 5 is a high-performance liquid chromatogram indicating an example of separation of a mixture according to a method of the invention.

[Fig. 6] Fig. 6 is a graph showing the relation between the blood sampling time and "% of dose" in a retentivity-in-blood test after injection of different types of liposome preparations having different rates of modification with PEG.

[Fig. 7] Fig. 7 is a graph showing the relation between the rate of modification with PEG of liposome preparations and corresponding AUC.

[Fig. 8] Fig. 8 is a graph showing the relation between the retention time of liposome preparations with different rates of modification with PEG of high-performance liquid chromatogram and AUC corresponding to the liposome preparations with the different rates of PEG modification.

Best Mode for Carrying Out the Invention

Closed vesicles which are separated by the separation method of the invention and membrane-modified with a hydrophilic polymer, preparations containing a drug therein and methods for preparing same are particularly described. In the invention, modification or membrane modification means a state wherein a hydrophilic polymer is fixedly held chemically, physically or electrically on an outer surface of a membrane forming a closed vesicle. In the practice of the invention, the closed vesicle is not critical so far as it is a particulate membrane structure (carrier) capable of encapsulating a drug therein, and generally contemplates to widely include, aside from a closed vesicle serving as a W/O/W carrier, a micell, a microsphere and the like classified as a W/O carrier. It will be noted that W means an aqueous phase and O means an oil phase.

The closed vesicle includes, for example, a liposome, a mirocapsule, a lipid microsphere, a nonoparticle or the like in particular. These may take a spherical form or a form close thereto.

The particle size (an outer diameter of the particle) of the closed vesicle may differ depending on the type thereof and is generally at 0.01 to 500 $\mu$m. For instance, with the liposome, the size is generally at 0.02 to 1 $\mu$m, preferably at 0.05 to 0.25 $\mu$m. With the microcapsule, the size is generally at 1 to 500 $\mu$m, preferably 1 to 150 $\mu$m. With the lipid microsphere, the size is generally at 1 to 500 $\mu$m, preferably at 1 to 300 $\mu$m. The size of a nanoparticle is generally at 0.01 to 1 $\mu$m, preferably at 0.01 to 0.2 $\mu$m.

The above-indicated particle size is measured by a dynamic light scattering method and is obtained as an average value of diameters of whole particles.

The closed vesicle in the invention should preferably have a latent function to encapsulate a drug therein at high concentration. Of the above-indicated vesicles, the liposome is preferred. In the practice of the invention, one wherein a drug is contained in the closed vesicle is called preparation.

The closed vesicle is generally formed, as a membrane material, of an amphipatic lipid containing a hydrophobic group and a hydrophilic group. The liposome is mainly illustrated hereinbelow by way of example.

A liposome is a closed vesicle generally formed with a lipid-bilayer membrane mainly composed of a phospholipid. More specifically, a liposome is one wherein a phospholipid having a hydrophobic group and a hydrophilic group forms a membrane in an aqueous solvent based on both polarities thereof and has a closed space structure formed by the membrane. The liposome is usually dealt with a state of suspension wherein an inner aqueous phase and an outer aqueous phase within the closed space exist as kept away from each other through the membrane.

It will be noted that in the present specification, the term "liposome" is intended to mean including this liposome suspension. To encapsulate or internally include a drug in a liposome means that a drug is contained as an inner aqueous phase or in an inner aqueous phase, and a liposome preparation means a liposome encapsulating (internally including) the drug therein. The encapsulation (inclusion) means including a drug held such as by attachment to the membrane or held in the membrane. In this case, the drug does not always exist in the inner aqueous phase.

The phospholipid is generally an amphipatic substance that has a hydrophobic group constituted of a long-chain alkyl group and a hydrophilic group constituted of a phosphate group in the molecule. Examples of the phospholipid include:

glycerophospholipids such as phosphatidylcholin

(lecithin), phosphatidyl glycerol, phosphatidic acid, phosphatidyl ethanolamine, phosphatidylserine, phosphatidyl inositol and the like; sphingophospholipids such as sphingomyelin and the like; natural or synthetic phosphatidyl phospholipids and derivatives thereof such as cardiolipin; and partially or fully hydrogenated products thereof (e.g. hydrogenated soybean phosphatidylcholin (HSPC)) and the like.

Of these, hydrogenated phospholipids such as hydrogenated soybean phosphatidylcholin and sphingomyelin are preferably used.

The liposome may be one which contains, as a main membrane material, one or a plurality of above-indicated phospholipids.

In order that an encapsulated drug is not readily leaked out during storage or in the living body such as blood or taking into account the case where the liposome is exposed to a temperature higher than the living body temperature (typically about 60°C) during the course of preparation, it is convenient to use, as a main membrane material, a phospholipid whose phase transition point is higher than an in vivo temperature (35 to 37°C). One of preferred embodiments is that the phase transition point of the main membrane material for liposome is not lower than 50°C.

The liposome may contain, aside from the phospholipid, other types of membrane constituent components. Other membrane constituent components include, for example, lipids other than phospholipids and derivatives thereof (which may be sometimes referred to hereinafter as "other lipids"). Lipids other than phospholipids are those lipids which have a hydrophobic group constituted such as of a long-chain alkyl group in the molecule and is free of a phosphate group therein. The lipids are not critical in type. Mention is, for example, of glyceroglycolipids, sphingoglycolipids, sterols such as cholesterol, and derivatives such as hydrogenated products thereof. Sterols are not critical so far as they have a cyclopentanohydrophenanthrene ring. For instance, cholesterol is mentioned. These other glycolipids may be contained singly or in plurality.

The phospholipid is generally contained in 20 to 100 mol%, preferably 40 to 100 mol% in the total lipids constituting the liposome membrane. Other lipids are generally in the range of 0 to 80 mol%, preferably 0 to 60 mol%, of the total lipids.

Of these, a liposome formed of a membrane of a mixed lipid of the above-mentioned phospholipid and other lipids is a preferred one.

The lipid bilayer membrane structure of a liposome may be in the form of either a unilamellar vesicle or a multilamellar vesicle (MLV). The unilamellar vesicle may be either SUV (small unilamellar vesicle) or LUV (large unilamellar vesicle). From the standpoint of liposome stability, LUV having a particle size of 0.05 to 0.25 $\mu$m is preferred.

The hydrophilic polymer modifying such a lipid membrane therewith is not critical in type and known hydrophilic polymers may be mentioned. Specific examples of the hydrophilic polymer include polyethylene glycols, polyglycerines, polypropylene glycols, ficol, polyvinyl alcohol, styrene-maleic anhydride alternate copolymer, divinyl ether-maleic anhydride alternate copolymer, polyvinylpyrrolidone, polyvinyl methyl ether, polyvinyl methyloxazoline, polyethyloxazoline, polyhdyroxypropyloxazoline, polyhydroxypropyl methacrylamide, polymethacrylamide, polydimethylacrylamide, polyhydroxypropyl methacrylate, polyhydroxyethylacrylate, hydroxymethyl cellulose, hydroxyethyl cellulose, polyaspartamide, synthetic polyaminoic acids and the like. Moreover, there may be mentioned water-soluble polysaccharides such as glucuronic acid, sialic acid, dextran, pullulan, amylose, amylopectin, chitosan, mannan, cyclodextrin, pectin, carrageenan and the like, and derivatives thereof such as, for example, glycolipids.

Of these, preferred hydrophilic polymers should preferably contain at least one unit selected from the group consisting of $-CH_2CH_2O-$, $-CH_2CH_2CH_2O-$, $-CH_2CH(OH)CH_2O-$ and $-CH_2CH(CH_2OH)O-$. The hydrophilic polymer may be a homopolymer wherein one of these units is repeated or a copolymer containing two or more of these units. The copolymer may take any form of a random copolymer, a block copolymer, a terpolymer or the like.

Of these, a homopolymer is preferred and especially, it is preferred to use polyethylene glycol (PEG), polyglycerine (PG) or polyprpylene glycol (PPG) that has been confirmed to show an effect of improving retentivity in blood of a liposome preparation.

These hydrophilic polymers are not critical with respect to the molecular weight thereof so far as they have such a molecular weight ensuring a molecular chain capable of developing an improving effect of retentivity in blood of a liposome preparation. The molecular weight of PEG generally ranges 500 to 10,000 daltons, preferably 1,000 to 7,000 daltons and more preferably 2,000 to 5,000 daltons. The molecular weight of PG generally ranges 100 to 10,000 daltons, preferably 200 to 7,000 daltons and more preferably 400 to 5,000 daltons. The molecular weight of PPG generally ranges 100 to 10,000 daltons, preferably 200 to 7,000 daltons and more preferably 1,000 to 5,000 daltons.

The hydrophilic polymer is generally used as a lipid derivative for membrane modification. It will be noted that although a hydrophilic polymer has usually a hydroxyl group at molecular terminals thereof, the terminal end side of the hydrophilic polymer, which is not bonded to a lipid of the lipid derivative, may be alkoxylated (e.g. methoxylated, ethoxylated or propoxylated) from the standpoint of preservation stability. In the separation method of the invention, separation is possible when the molecular terminals of the hydrophilic polymer are made of an alkoxy group.

The lipid forming a lipid derivative of a hydrophilic polymer is not critical in type so far as it is made of a compound having a hydrophobic moiety ensuring affinity for a liposome membrane. In general, mention is made of lipids such as phospholipids, sterols and the like, which have the same as or are analogous to the membrane component, long-chain fatty alcohols, polyoxypropylene alkyl, glycerine fatty acid esters and the like. Of these, phospholipids are preferred ones.

The phospholipids are not critical in type so far as they are able to bond to the above-mentioned hydrophilic polymers and include, for example, phosphatidyl ethanolamine, phosphatidyl glycerol, phosphatidylserine and the like. Of these, phosphatidyl ethanolamine is a preferred one. The acyl chain present in the phospholipid is favorably a saturated fatty acid having chain length of $C_{14}$-$C_{20}$, preferably $C_{16}$-$C_{18}$. The acyl chain includes, for example, dipalmitoyl, distearoyl, palmitoylstearoyl or the like.

The combination of a hydrophilic polymer and a lipid in the lipid derivative of the hydrophilic polymer is not critical. For instance, where PEG is used as a hydrophilic polymer, mention is made, as preferred, of a phospholipid or a cholesterol derivative. Such lipid derivatives of hydrophilic polymers can be prepared according to known methods, or some may be available as a commercial product. For example, distearoyl phosphatidyl ethanolamine derivatives of PEG (PEG-DSPE) is a general-purpose compound which is readily available.

The liposome (closed vesicle) used in the invention may be membrane-modified with one or two or more of lipid derivatives of the above-mentioned hydrophilic polymers.

The rate of liposome modification with a hydrophilic polymer is generally 0.1 to 10 mol%, preferably 0.1 to 5 mol% and more preferably 0.2 to 3 mol% when expressed as a ratio of the hydrophilic polymer to the lipid membrane (total lipids). It is to be noted that the total lipids include a lipid in the lipid derivative of the hydrophilic polymer.

The state of the membrane modification with the above-mentioned hydrophilic polymer may be mainly classified into categories including one wherein it is randomly distributed internally and externally of the membrane and one wherein it is selectively distributed outside the membrane surface. In the practice of the invention, the latter is preferred. Although the preparation method for the latter category will be described hereinlater, a liposome wherein a hydrophilic polymer is distributed selectively outside the membrane surface is preferred because such a liposome is able to develop its effect when using the hydrophilic polymer in an amount of half of a liposome wherein the hydrophilic polymer is randomly distributed internally and externally of the membrane and is advantageous thereover because of a less influence on the stability of the lipid membrane due to the efficiency of existence and the existence thereof, along with the fact that the hydrophilic polymer is unlikely to suffer an influence of a pH of an inner aqueous phase thereby ensuring the stability of the liposome preparation. When taking the separation effect obtained by the separation method of the invention into consideration along with the efficiency of modification in the membrane modification with such a hydrophilic polymer and the preparation stability, it is preferred that the liposome preparation in the present invention is such that an outer side of the former membrane is selectively modified with the hydrophilic polymer.

In the practice of the invention, other ingredients may be arbitrarily contained so far as they are able to keep the membrane structure composed of such membrane components as set out hereinabove and a liposome allows them to be contained therein. More particularly, mention is made, for example, of surface modifying agents other than hydrophilic polymer derivatives, such as compounds having a basic functional group, and charging materials such as an acidic functional group. The charging material is usually in the form of a lipid derivative. As a lipid, there are mentioned ones similar to lipid derivatives of hydrophilic polymers.

The basic functional group includes, for example, an amino group, a amidino group, a guadinino group and the like, and examples of compounds having these functions or lipid derivatives thereof (cationized lipids) include DOTMA set out in Japanese Patent Laid-Open No. Sho 61-161246, DOTAP set out in JP-T-Hei 5-508626, tansfectam (Transfectam) set out in Japanese Patent Laid-Open No. Hei 2-292246, TMAG disclosed in Japanese Laid-open patent Application No. Hei 4-108391, 3,5-dipentadesiloxybenzamidine hydrochloride, DOSPA, DOTAP, TfxTM-50, DDAB, DC-CHOL and DMRIE set out in PCT Patent Publication Pamphlet No. 97/42166, and the like.

Examples of the compound having an acidic functional group include: acidic phospholipids such as phosphatidic acid, phosphatidylserine, phosphatidylinositol, phosphatidylglycerol, cardiolipin and the like; saturated or unsaturated fatty acids such as oleic acid, stearic acid and the like; gangliosides having sialic acid such as gangliosideGM1, gangliosideGM3 and the like; and acidic amino acid surface active agents such as N-acyl-$_L$-glutamine and the like.

In the invention, drugs contained in such a liposome (closed vesicle) as set forth above are not critical in type so far as they are ones used for treatment and/or diagnosis. The drugs include, for example, nucleic acid, polynucleotides, genes and analogs thereof, anticancer drugs, antibiotics, enzyme drugs, antioxidant agents, lipid intake inhibitors, hormones, anti-inflammatory agents, steroid drugs, vasodilating agents, angiotensin-converting enzyme inhibitors, angiotensin-receptor antagonists, smooth muscle cell proliferation and migration inhibitors, antiplatelet aggregation drugs, anticoagulants, chemical mediator liberation inhibitors, endothelial cell growth promoters or inhibitors, aldose reductase inhibitors, mesangial cell proliferation inhibitors, lipoxygenase inhibitors, immunosuppresants, immunostimulators, antiviral drugs, Maillard reaction inhibitors, amyloidosis inhibitors, nitrogen monoxide synthesis inhibitors, AGEs (advanced glycaton endproducts) inhibitors, radical scavengers, proteins, hemoglobin solution, peptides, glycosaminoglycans and derivatives thereof, oligosaccharides and polysaccharides and derivatives thereof, and X-ray contrast agents, ultrasonic wave contrast agents, radiolabelling nuclear medicine diagnostic agents, diagnostic agents for nuclear magnetic resonance diagnosis and the like.

The liposome preparation of the invention may further contain pharmaceutically acceptable stabilizers and/or antioxidants although depending on the administration route. The stabilizer is not critical and includes, for example, a sugar such as glycerol, mannitol, sorbitol, lactose or sucrose. The antioxidant is not critical and includes, for example ascorbic acid, uric acid, $\alpha$, $\beta$, $\gamma$ or $\delta$ tocopherol analog (e.g. vitamin E).

The suspension medium, i.e. an outer aqueous phase, of the liposome preparation usually in the form of a suspension is not critical so far as it is pharmaceutically acceptable, depending on the administration route. Mention is made, for example, water, physiological saline solution, pharmaceutically acceptable organic solvents, aqueous solutions of collagen, polyvinyl alcohol, polyvinylpyrrolidone, carboxyvinyl polymer, sodium carboxymethylcellulose, sodium polyacrylate, sodium alginate, water-soluble dextran, sodium carboxymethyl starch, pectin, methylcellulose, ethylcellulose, xanthan gum, gum arabic, casein, gelatin, agar, diglycerine, propylene glycol, vaseline, paraffin, stearyl alcohol, stearic acid, human serum albumin (HSA), PBS, bioerodible polymers and the like, and serum-free media.
Of these, water and physiological saline solution are preferred. These media may contain a surface active agent acceptable as a pharmaceutical additive or a buffer agent with a physiological pH acceptable in the living body.

The liposome (closed vesicle) of the type wherein the membrane is modified with such a hydrophilic polymer as set out hereinabove and liposome preparations can be prepared according to known procedures. The liposome (closed vesicle) of the type wherein a hydrophilic polymer is selectively distributed at an outer side of the membrane surface can be obtained by a two-stage procedure wherein a liposome has been once formed, after which a lipid derivative of a hydrophilic polymer is added for modification. As to the liposome forming method per se, a variety of techniques are known including, for example, an ethanol injection method, a thin film method, a reverse-phase evaporation method, a freeze thaw method, a ultrasonic wave method, a high pressure discharge method (see "Liposomes in Life Science" edited by Terada, Yoshimura, etc.; Springer-Verlag Tokyo Inc. (1992), which is incorporated herein by reference), an extrusion method and the like. Using such lipids serving as a membrane constituting component as set out above, one of the above methods is appropriately adopted thereby enabling a liposome to be prepared.

Furthermore, a known sizing technique (e.g. edited by G. Gregroriadis "Liposome Technology Loposome Preparation and Related Techniques" 2nd edition, Vol. I-III, CRC Press (which is incorporated herein by reference) is applied to thereby obtaining a liposome having a desired size.
A unilamellar method of liposome is also known (e.g. in the above-mentioned document ("Liposomes in Life Science")) and may be appropriately adopted.

Next, a hydrophilic polymer capable of modifying a liposome therewith, such as a lipid derivative, is added to the thus formed liposome, so that the hydrophilic polymer can be selectively distributed at the outer side of the liposome membrane.
The drug may be encapsulated by appropriately adopting a known introducing method in an appropriate step depending on the type of drug, i.e. in the step of forming the liposome, after the liposome forming step or after the membrane modifying step.
After the introduction of the drug, the drug, which is left not encapsulated in the liposome, is usually removed by a known removing method.

The separation method of the invention is separation of closed vesicles whose membrane is modified with a hydrophilic polymer. Especially, in the practice of the invention, closed vesicles, typical of which is a liposome of a structure wherein the membrane surface is selectively modified at the outer side thereof with the hydrophilic polymer, can be directly separated. The hydrophilic polymer existing inside the closed vesicle cannot be evaluated according to this separation method and does not function as desired. The separation method of the invention is suitable for separation between the closed vesicles modified individually with the same kind of hydrophilic polymer. More particularly, closed vesicles having different rates of membrane modification are separated from one another by ion-exchange chromatography (which may be hereinafter abbreviated as IEC) using a concentration gradient wherein an ionic intensity of an eluent is changed with time.

IEC is a known technique to separate counterions from one another using an ion exchange resin chemically bonding molecules capable of dissociation on the surface of ion exchange resin particles and causing a charged surface to be formed, and is broadly classified into cation exchange and anion exchange depending on the surface charge state. For the anion exchange, it is usual to use resins into which a cationic residue such as quaternary ammonium, a diethylaminoethyl group or the like is introduced. For the cation exchange, resins into which an anionic residue, such as a sulfopropyl group, is introduced are usually used. In the invention, IEC using anion exchange is preferred and in particular, IEC using weak anion exchange such as with a diethylaminoethyl group is more preferred. The ion exchange resin may take the form of either porous particles or non-porous particles. Columns filled with these ion exchange resins are commercially available and can be used for the separation of the invention. Commercially available weak anion exchange resin columns include DEAE-5PW (made by TOSOH Corporation), YMC-Pack IES-AX (made by YMC Co., Ltd.) and the like.

IEC can be measured by mounting an appropriate one of these columns in a high-performance liquid chromatograph (HPLC). The measured data can be used for evaluation of drugs as will be described hereinafter.

In the practice of the invention, in view of the possibility that closed vesicles to be handled undergo solvent denaturation, an eluent that is completely free of an organic solvent is conveniently used. In IEC, an optimum pH range differs depending on the type of ion exchange resin and the charge state is influenced, for which it is desirable that the pH of the eluent (which may be hereinafter referred to as mobile phase) be appropriately controlled. Generally, from the standpoint of the nature of ion exchange resin, the pH of the eluent is preferably within a pH range of 2 to 10 and from the standpoint of the stability of closed vesicle, especially liposome, the pH is preferably at 4 to 10. With IEC using an anion exchange resin, the pH of the eluent is preferably at 6 to 10, more preferably at 6 to 9.

The eluent may be buffered, for example, with a phosphate buffer, a Tris hydrochloride buffer or an acetate buffer. The concentration of the buffer is generally at 10 to 50 mM.

In the invention, the concentration gradient is created in the eluent of IEC. For the creation of the concentration gradient, there are mainly used a method wherein the eluent is changed in solvent composition and a method wherein an ionic intensity is changed with time.

In view of the possibility that closed vesicles undergo solvent denaturation, the method of changing an ionic intensity with time is convenient in the invention.

In the practice of the invention, the ionic intensity is usually controlled by use of NaCl, ammonium sulfate or the like, of which NaCl is beneficially used in view of the ease in controlling the ionic intensity. It is desirable to select an ionic intensity within a range not influencing the structure of closed vesicle. More particularly, with the liposome, the selection of an ionic intensity not causing membrane permeation is preferred and particularly, the intensity is preferably not greater than 2 mols/L in maximum concentration when NaCl is used.

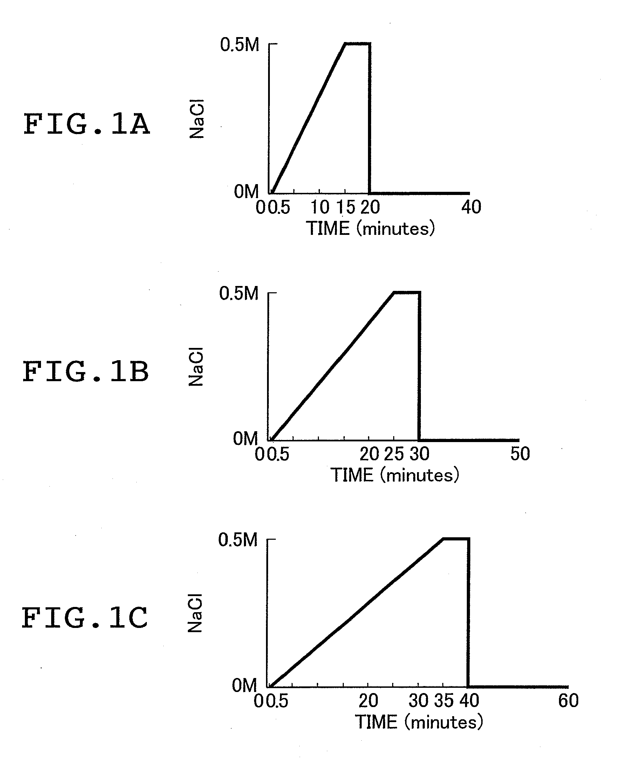

The concentration gradient method includes variations of a linear type wherein the ionic intensity changes at a given rate, of a curve type (gradient elusion of concave or convex type) and of a stepwise type wherein the ionic intensity is changed in a stepwise manner although they are not critical. In view of the ease in control, the linear type is preferred. The manner of making the ionic intensity with a concentration gradient of the linear type is feasible according to a known method. The linear-type concentration gradient is schematically shown in Fig. 1.

In the separation method of the invention, the rate of the concentration gradient is important. Under the same flow rate, a smaller gradient leads to more improved separability. This is particularly shown in examples appearing hereinafter.

For instance, in a separation instance of a PEG-modified liposome preparation by IEC to which a concentration gradient $(0 \rightarrow 0.5M)$ based on NaCl is applied, it has been confirmed that when the final ionic intensities are equal to each other, a lower rate of concentration gradient leads to more improve separability based on the rate of PEG modification. Especially, where a concentration gradient is brought about over 35 minutes, it has been confirmed that there can be obtained chromatograms, in which the respective separation peaks are distinctly separated from one another,'from liposome preparations having rates of PEG modification of about 0.5 mol%, about 0.25 mol% and also from a non-modified liposome preparation. More particularly, the liposome preparations having the different rates of PEG modification can be individually separated and collected as IEC fractions, so that a liposome preparation having a desired rate of PEG modification can be purified and isolated from a mixture whose rate is widely distributed. When the fraction time in the vicinity of an effluent time corresponding to a liposome preparation having a desired rate of modification with PEG is set as desired, the width of the distribution can be controlled.

It will be noted that although a preparation having a rate of PEG modification of not lower than 1 mol% is separable under conditions where the concentration gradient is made much lower than those of the above instance, the time required for the separation should be as short as possible when taking an actual operation efficiency into account. As illustrated in examples appearing hereinafter and described below, if liposome preparations having a rate of PEG modification of up to about 0.5 mol% can be purified, the main purpose of modification with PEG can be achieved from the knowledge on the effect of the rate of modification with PEG on the evaluation of the modification effect of PEG in liposome preparations. From this standpoint, separation is generally feasible in about 180 minutes at the longest.

The separation conditions in IEC are not critical except for the selection of the column as set out above, the type of solvent in the mobile phase and the use of concentration gradient and may be appropriately selected from ordinary IEC conditions. Although the flow rate of the mobile phase is usually at 0.05 to 0.1 ml/minute for a column whose diameter φ of 2.0 mm, this rate has to be set while taking a maximum pressure of a gel packed in the column into consideration.

In the present invention, according to the above separation method, the effect of modification of a closed vesicle (preparation) with a hydrophilic polymer can be more rigorously assessed over a conventional analyzing method of a rate of PEG modification of a liposome preparation (closed vesicle). Especially, the rate of surface modification of a closed vesicle (preparation) measured by the a conventional analyzing method is an average value of all the measured samples and if, for example, the surface modification rate is at 0.25 mol%, it is more to the point that such samples could not be discriminated from a mixture of those of 0 mol% and 0.5 mol%. In this case, the effect of the surface modification rate on the retentivity in blood cannot be exactly evaluated along with a difficulty in verification thereof. When using the separation method of the invention, separation is possible with respect to each surface modification rate and at least, it can be known how the surface modification of the prepared closed vesicles is distributed (or varied in width). In addition, since there can be obtained a preparation that has a desired surface modification rate and is highly purified by selection of a fraction width, the surface modification effect can be rigorously evaluated. For instance, an interrelation between a separated and purified liposome preparation and the results of a test (AUC) for the preparation can be seen. Moreover, the effect of the surface modification can be evaluated by relating the peaks of the respective preparation of IEC to corresponding AUSs.

When the separation method of the invention is applied to a manufacturing process of closed vesicles (or preparations) modified with a hydrophilic polymer, there can be obtained closed vesicles (preparations) highly purified at a desired modification rate. For instance, the separation method is applied as a purifying step of closed vesicles (preparations) whose membrane is modified with a hydrophilic polymer and which contain a drug therein. The closed vesicles (preparations) are separated depending on the rate of membrane modification with the hydrophilic polymer to collect closed vesicles (or preparations) having a desired rate of the hydrophilic polymer modification from the separated closed vesicles (preparations). According to the manufacturing process, there can be obtained closed vesicles modified with a hydrophilic polymer at the outer surface of the membrane thereof, which are free of those vesicles having such a modification rate with the hydrophilic polymer as to be small in dispositional effectiveness, more particularly, the vesicles having a hydrophilic polymer modification rate of less than 0.5 mol% relative to the membrane lipid of the closed vesicle. In the practice of the invention, it is easy to eliminate those having a modification rate of 0 mol%. Accordingly, the invention can provide closed vesicles (preparations) highly purified at such a desired modification rate. An embodiment wherein the membrane-modified closed vesicle is a liposome (or preparation) subjected to membrane modification with polyethylene glycol is a preferred one.

If the above separation method is applied to analysis of closed vesicles (or preparations) membrane-modified with a hydrophilic polymer and the resulting analysis data are fed back to the manufacturing process of the closed vesicles (or preparation), the manufacturing conditions of the manufacturing process can be controlled as having a desired modification rate thereby obtaining closed vesicles (or preparation) that are highly controlled at a desired hydrophilic polymer modification rate.

Examples

The invention is described in more detail by way of examples and should not be construed as limited to these examples and test examples.

(Preparatory Example 1)

(1) Preparation of liposome

10 ml of absolute ethanol (made by Wako Pure Chemical Industries, Ltd.) was added to 7.021 g of hydrogenated soybean phosphatidylcholine (HSPC)(with a molecular weight of 790, SPC3 made by Lipoid AG) and 2.927 g of cholesterol (Chol)(with a molecular weight of 386.65, made by Solvay Co.) and heated to 68°C for complete dissolution. Thereafter, 90 ml of a 250 mM ammonium sulfate (made by Wako Pure Chemical Industries, Ltd.) aqueous solution, followed by swelling in a thermostatic bath of 68°C for 15 minutes and vortex agitation to prepare a liposome crude dispersion. Next, the dispersion was successively passed through filters (0.2 $\mu$m in pore diameter $\times$ 5 times and 0.1 $\mu$m $\times$ 10 times, made by Whatman K.K.) attached to an extruder (The Extruder T.100, made by Lipex Biomembranes Inc.) to obtain a liposome dispersion (which may be hereinafter referred to simply as liposome).

(2) PEG modification

Polyethylene glycol (molecular weight of 5000)-phophatidylethanolamine (PEG$_{5000}$-DSPE) (molecular weight of 6075, made by NOF Corporation) was diluted with distilled water to prepare a PEG$_{5000}$-DSPE aqueous solution with a concentration of 36.74 mg/ml.
The PEG$_{5000}$-DSPE aqueous solution was added to individual containers containing the liposome divided into 8 ml portions in theoretical amounts corresponding to given concentrations of 0 (liposome preparation 1 described hereinafter), 0.25 (liposome preparation 2), 0.5 (liposome preparation 3), 0.75 (liposome preparation 4), 1 (liposome preparation 5) or 2 (liposome preparation 6) mol% with respect to the PEG modification rate (mol%) calculated by (PEG$_{5000}$-DSPE/ total lipids) $\times$ 100, followed by agitation at 60°C for 30 minutes and cooling with ice.

(3) Substitution of outer aqueous phase

Using a column (Sepharose 4FF) substituted with 10 mM Tris (pH 9.0) 10% sucrose, the thus obtained PEG-modified liposomes were subjected to gel filtration to substitute the outer aqueous phase.

(4) Drug introduction

The samples obtained after the gel filtration were subjected to quantitative analysis of HSPC concentration (mg/ml) by use of a phospholipid quantitative determination kit (phospholipid C Test Wako (choline determination), made by Wako Pure Chemical Industries, Ltd.). Based on this HSPC concentration, the total lipid quantity (mM) was calculated. The total lipid quantity used herein means a total amount of HSPC, Chol and DSPE.
Doxorubicin hydrochloride (Dox: molecular weight of 579.99) was added to 10 ml of 10 mM Tris (pH 9.0) 10% sucrose in such amounts that Dox/Total Lipid (mol/mol) = 0.16 relative to the total lipid weight, respectively, which were added to the respective PEG-modified liposomes each subjected to the outer aqueous phase substitution, followed by heating at 60°C for 60 minutes while agitating to introduce the Dox.

(5) Aftertreatments

The liposome after the drug introduction was cooled with ice and subjected to gel filtration through a gel column (Sepharose 4FF) substituted with 10 mM histidine (pH 6.5) 10% sucrose to eliminate the drug left unloaded in the liposome. Next, sterilization treatment was carried out by passage through a sterilizing filter (Minisart Plus 0.2 $\mu$m) to obtain PEG-modified doxorubicin encapsulated liposomes (hereinafter referred to liposome preparations) 1 to 6.

(6) Analyses

<PEG modification rate>

The respective liposome preparations obtained above were subjected to quantitative analysis of a concentration (mg/ml) of the lipids (HSPC, Chol and PEG$_{5000}$-DSPE) according to high performance liquid chromatography (column: porous silica gel chemically bonded with a phenyl group, mobile phase: buffer/MeOH/EtOH mixed solution) using a differential refractometer as a detector. The total lipid concentration (total lipids/mM) and PEG modification rate (mol%), both calculated from the resulting analyzed value, are shown in Table 1 along with the theoretic mol% of the PEG$_{5000}$-DSPE charge. The measurements of the PEG modification rate obtained herein are, respectively, an average value of all of the measured samples.

<Drug concentration>

The concentration (mg/ml) of doxorubicin hydrochloride (Dox) was quantitatively analyzed according to a calibration method based on the absorbance at 480 nm. The calibration curve sample used was a physiological saline solution containing Dox at given concentrations (mg/ml). The absorbance was measured by dispersing 40 $\mu$l of the sample in 2 ml of methanol for fluorescent analysis.
From the Dox concentrations, the mM calculation values and molar ratios (Dox/Total lipids) relative to total lipids of the respective liposome preparations were calculated. These values are shown in Table 1.

<Particle size>

20 ml of each liposome preparation was diluted with 3 ml of physiological saline solution to measure an average particle size (nm) by means of Zetasizer 3000HS (made by Malvern Instruments.). The results are shown in Table 1.
[Table 1]

| Liposome preparation | Total lipids (mM) | PEG modification rate (mol%)* | | Dox concentration | | Dox/total lipids (mol/mol) | Particle size (nm) |
|---|---|---|---|---|---|---|---|
| | | Charge amount | Measurement | (mg/ml) | (mM) | | |
| 1 | 34.7 | 0 | 0 | 2.6 | 4.6 | 0.13 | 124.1 |
| 2 | 25.1 | 0.25 | 0.27 | 2 | 3.5 | 0.14 | 119.5 |
| 3 | 23 | 0. 5 | 0.54 | 1.8 | 3.2 | 0.14 | 125.5 |
| 4 | 26.1 | 0.75 | 0.78 | 2 | 3.5 | 0.13 | 128.7 |
| 5 | 24 | 1 | 1.08 | 1.8 | 3.2 | 0.13 | 128.2 |
| 6 | 25.3 | 2 | 2.14 | 2.2 | 3.9 | 0.15 | 132.7 |

\* PEG modification rate (mol%) = (PEG$_{5000}$-DSPE/total lipids) × 100

(Example 1)

Using a high performance liquid chromatograph (HPLC) equipped with an anion exchange column, the liposome preparations 1 to 6 prepared in Preparatory Example 1 were subjected to measurement by application of a NaCl concentration gradient method in the mobile phase. The measuring conditions are indicated below.

Mobile phase: the NaCl concentration in 20 mM Tris (pH 9.0) was changed in the range of 0 M to 0.5 M under the following three conditions 1 to 3. More particularly, the NaCl concentration in 20 mM Tris was subjected to linear concentration gradient ($0 \rightarrow 0.5$ M) from the commencement of effluence of 0.5 minutes till the time indicated below, followed by keeping a 0.5 M NaCl concentration for 5 minutes. Thereafter, NaCl-free 20 mM Tris (pH 9.0) was run off for 20 minutes. These conditions are shown in Fig. 1 (A: conditions 1, B: conditions 2, C: conditions 3).

Conditions 1: 0.5 to 15 minutes ($0 \rightarrow 0.5$ M) + 5 minutes (0.5 M) + 20 minutes (0 M)
Conditions 2: 0.5 to 25 minutes ($0 \rightarrow 0.5$ M) + 5 minutes (0.5 M) + 20 minutes (0 M)
Conditions 3: 0.5 to 35 minutes ($0 \rightarrow 0.5$ M) + 5 minutes (0.5 M) + 20 minutes (0 M)

Flow rate: 1 ml/minute

Column: DEAE-5PW (column: diameter $\varphi$ of 2.0 mm and length of 75 mm) (porous hydrophilic polymer gel column for anion exchange HPLC, made by TOSOH Corporation) was used after having been preliminarily substituted with 20 mM Tris (pH 9.0).

Column temperature: not critically set (ambient temperature of 28 to 32°C)

Injection amount: 10 $\mu$l (measurement under the above conditions 3 was also made at 5 $\mu$l and 20 $\mu$l to confirm peak positions same as at 10 $\mu$l (not shown)).

Measuring absorption wavelength: 254 nm (conditions 1) or 280 nm (conditions 2 and 3)

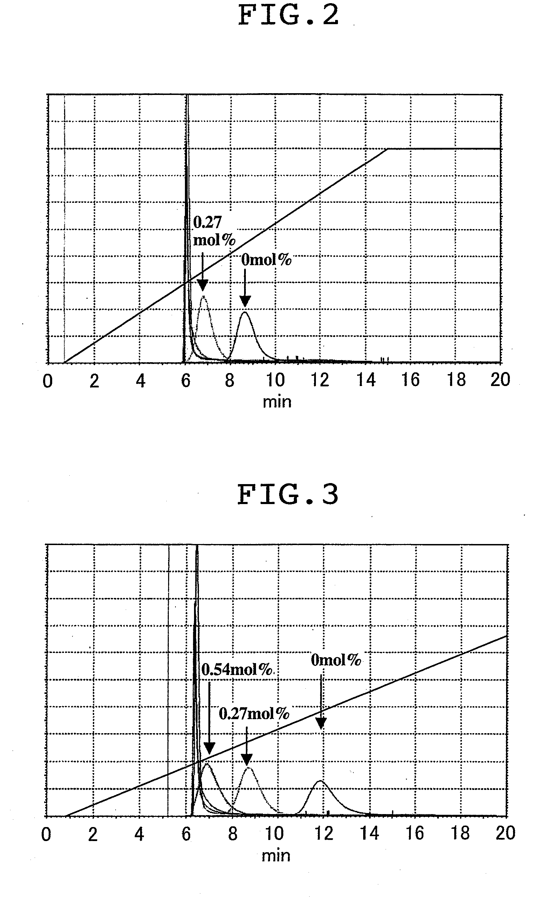

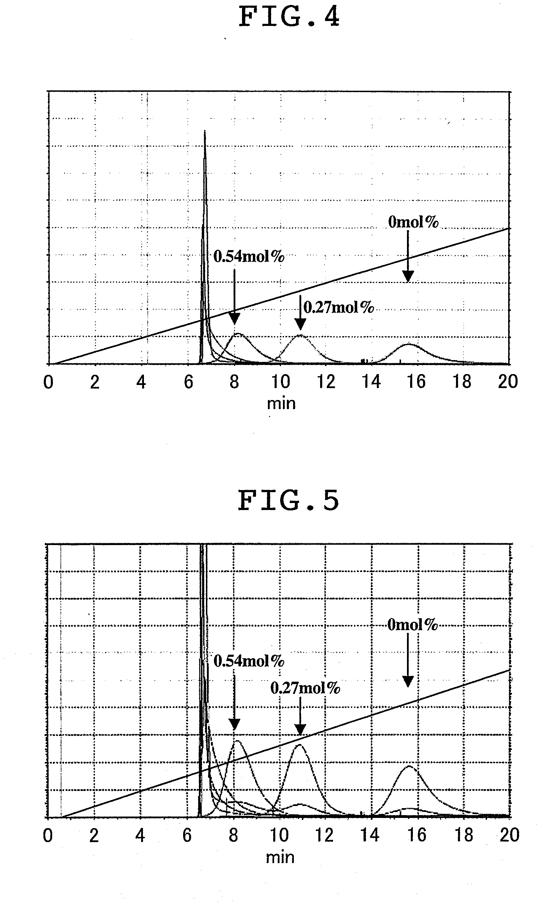

The liposome preparations 1 to 6 having different rates of PEG modification prepared in Preparatory Example 1 were subjected to measurements under the respective NaCl concentration conditions. Individual measured charts of the same NaCl concentration conditions were mutually superposed on the papers and charts obtained by combining datas of the respective conditions are shown in Fig. 2 (conditions 1), Fig. 3 (conditions 2) and Fig. 4 (conditions 3).

As shown in these figures, it has been found that the liposome preparations differ in effluence time depending on the PEG modification rate by application of the NaCl concentration gradient method. More particularly, it has been found that the PEG-modified liposome preparations can be separated by the difference in the PEG modification rate. Moreover, it has become apparent that the separability is enhanced when the NaCl concentration gradient is made gentle. Especially, with liposome preparation modified with $PEG_{5000}$ on the surface thereof, high-accuracy separation is possible up to a modification rate of 0.5 mol%. This value of 0.5 mol% is an dynamically effective modification rate of the liposome as will be shown in Test Example appearing hereinafter, meaning that the method of the invention is very useful in the manufacture of PEG-modified liposome preparations.

According to the above-mentioned method by which preparations having modification rates of not greater than 0.5 mol% is easily separated, when collecting those having a modification rate of not less than 0.5 mol% without collection of those whose rate is not greater than 0.5 mol%, high purification is possible only to ones having a dynamically effective modification rate of liposome. The theoretical modification rate of the added $PEG_{5000}$-DSPE and the maximum modification rate are substantially equal to each other. Therefore, in case where it is added so as to make, for example, 1 mol%, collection of ones having modification rates of not less than 0.5 mol% without containing ones whose rate is less than 0.5 mol% allows a liposome preparation whose modification rate is within $0.75 \pm 0.25$ mol% to be obtained.

(Example 2)

Equivalent amounts of the PEG-modified liposome preparations 1 to 6 prepared in Preparatory Example 1 were taken, respectively, and mixed together. This mixture sample was applied with the NaCl concentration gradient conditions 3, followed by measurement with liquid chromatography, like Example 1. The results are shown in Fig. 5.

As shown in Fig. 5, the liposome preparation mixture could be separated from one another depending on the difference in the PEG modification rate. The respective peak positions were coincident with those positions indicated in Fig. 4. From this, it has been confirmed that the method of the invention enables liposome preparations having different modification rates to be mutually separated from one another from the mixture of the liposome preparations having the different modification rates.

(Test Example 1)

The liposome preparations 1 to 6 (10 $\mu$mols/2 ml/kg as a total lipid weight) were administered to mice from the tail vein thereof to check the transitional level of blood concentration (each group including 3 mice).

1, 3, 6, 24 and 48 hours after the administration, about 0.5 ml of the blood was sampled from the tail vein by use of a 25G bladed intravenous needle (Terumo Corporation) added with hepalin (1000 units/ml, Aventis Pharmer) and a 1 ml injection syringe (Terumo Coporation) for tuberculin. The blood was placed on an ice bath and subjected to centrifugal separation (Beckman GS-15R, 10000 r.p.m., 4°C, 10 minutes) after completion of the blood sampling to separate the blood plasma. The doxorubicin concentration in the plasma was determined by measuring a fluorescence intensity at excitation wavelength (Ex) = 500 nm and fluorescence wavelength (Em) = 580 nm.

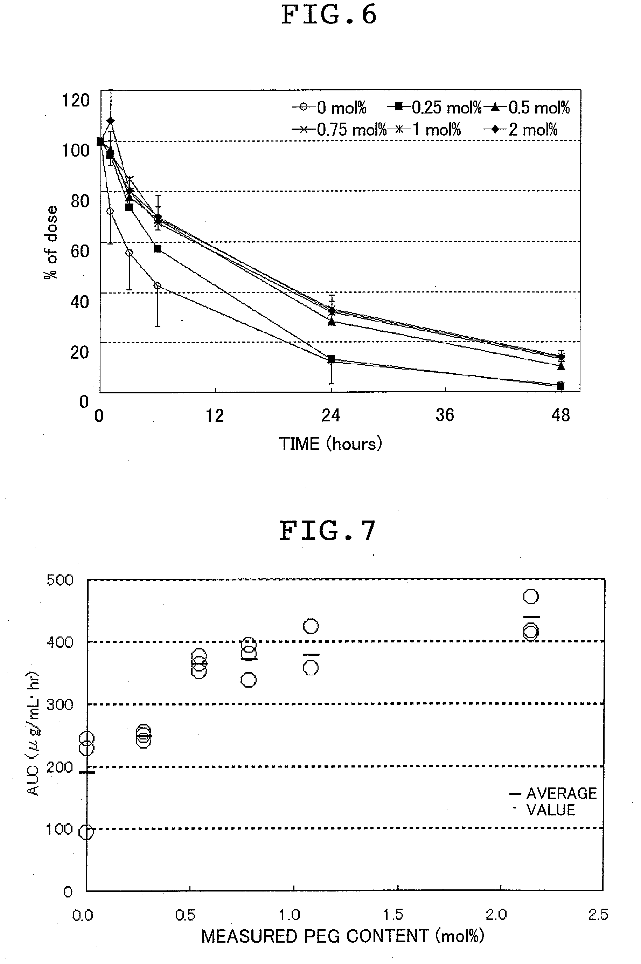

The residual ratio (%) relative to a dose at the time of the respective blood sampling after the injection was obtained while approximately calculating a residual ratio at the time of the administration (0) as 100%. The "% of dose" relative to the lapse of time is shown in Fig. 6.

As shown in Fig. 6, the liposome, not introduced with PEG, is lower in blood concentration than other liposomes at the initial stage of administration and is not higher than 2/3 of other liposomes (after a lapse of 6 hours). Although a higher PEG modification rate (mol%) results in higher retentivity in blood, the blood concentration is substantially kept unchanged when exceeding 0.5 mol%. The "% of dose" at 24 hours was about 30% and the "% of dose" at 48 hours was about 17%. From this, the effect of the PEG modification is satisfactorily obtained at a PEG modification rate of about 0.5 mol%.

The PEG modification rate was evaluated from the standpoint of AUC (area under the curve). Fig. 7 shows AUC based on the absorption factor obtained from the blood concentration after the injection time relative to the PEG modification rate (measurement) of the respective liposome preparations.

It will be noted that AUC = X(absorption factor)/$V_d$ (distribution volume) x $K_{el}$ (elimination rate)

The denominator factors $V_d$ and $K_{el}$ are determined by the type of drug and AUC is regarded in proportion to the absorption factor.

Like the results shown in Fig. 6, it has been found that AUC increases with an increase in PEG modification rate up to a PEG modification rate of 0.5 mol%. When the PEG modification rate exceeds 0.5 mol%, the increase of AUC is not significant and is saturated substantially at 0.5 mol%.

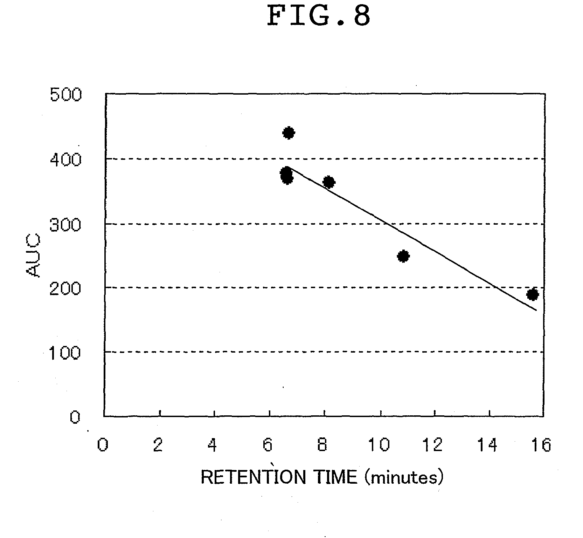

In Fig. 4, the above-determined values (average values) of AUC (see Fig. 7) corresponding to the respective PEG modification rates are applied to the peak positions of the respective PEG modification rates (time axis) to check the relation between the AUC and the retention time in the high-performance liquid chromatogram. This is shown in Fig. 8. As shown in Fig. 8, there is a negative interrelation between the AUC and the retention time in the high-performance liquid chromatogram.

From these results, it has been made clear that the method of the invention is able not only to analyze the PEG modification rate on the surface of a liposome preparation, but also to physicochemically confirm the retentivity in blood of the liposome preparation.

## Claims

1. A method for separating closed vesicles membrane-modified with a hydrophilic polymer, the method comprising separating closed vesicles differing in rate of membrane modification by use of ion-exchange chromatography applied with a concentration gradient wherein an ionic intensity of an eluent is changed with time.

2. The separation method according to claim 1, wherein said hydrophilic polymer is made of polyethylene glycol and the closed vesicle is made of a liposome.

3. The separation method according to claim 1 or 2, wherein the ionic intensity is an ionic intensity of NaCl.

4. The separation method according to any of claims 1 to 3, wherein the ion-exchange chromatography is a weak anion-exchange chromatography.

5. The separation method according to claim 4, wherein said eluent is an aqueous solvent containing a buffer agent and having a pH of 6 to 10.

6. The separation method according to any of claims 1 to 5, wherein said closed vesicle is a preparation containing a drug in the closed vesicle.

7. A method for preparing closed vesicles, the method comprising:

   providing closed vesicles subjected to membrane modification with a hydrophilic polymer;
   separating the closed vesicles depending on the rate of membrane modification with the hydrophilic polymer by the separation method of the closed vesicles defined in any of claims 1 to 6; and

collecting closed vesicles having a desired rate of modification with the hydrophilic polymer to obtain purified closed vesicles.

8. The preparation method according to claim 7, wherein the membrane-modified closed vesicle is a liposome whose membrane is modified with polyethylene glycol.

9. Closed vesicles modified with a hydrophilic polymer on an outer surface of a membrane thereof, which are free of closed vesicles wherein a rate of modification with the hydrophilic polymer is less than 0.5 mol% relative to a membrane lipid of the closed vesicle.

10. The closed vesicles according to claim 9, wherein the hydrophilic polymer is made of polyethylene glycol and the closed vesicle is made of a liposome.

11. The closed vesicles according to claim 9 or 10, wherein the closed vesicle is a preparation containing a drug therein.

12. A method for evaluating a preparation of a closed vesicle membrane-modified with a hydrophilic polymer and containing a drug therein on the basis of a rate of modification with the hydrophilic polymer by use of the separation method defined in any of claims 1 to 6.

FIG.1A

FIG.1B

FIG.1C

# FIG.2

# FIG.3

# FIG.4

# FIG.5

# FIG.6

# FIG.7

# FIG.8

## INTERNATIONAL SEARCH REPORT

International application No.

PCT/JP2007/063287

A. CLASSIFICATION OF SUBJECT MATTER
*A61K9/127*(2006.01)i, *A61K47/24*(2006.01)i, *A61K47/28*(2006.01)i, *A61K47/34* (2006.01)i, *B01D15/04*(2006.01)i, *G01N30/02*(2006.01)i, *A61K31/704* (2006.01)n, *A61P35/00*(2006.01)n
According to International Patent Classification (IPC) or to both national classification and IPC

B. FIELDS SEARCHED

Minimum documentation searched (classification system followed by classification symbols)
A61K9/127, A61K47/24, A61K47/28, A61K47/34, B01D15/04, G01N30/02, A61K31/704, A61P35/00

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| P,X<br>P,A | WO 2005/092388 A1 (TERUMO CORP.),<br>06 October, 2005 (06.10.05),<br>Full text; particularly, Claim 12; Par. Nos.<br>[0035], [0069] to [0074] (example 1);<br>Par. Nos. [0078] to [0081] (example 2)<br>& EP 1731172 A1     & CN 1938048 A<br>& KR 20070011331 A | 9-11<br>1-8,12 |
| P,X<br>P,A | JP 2006-273812 A (TERUMO CORP.),<br>12 October, 2006 (12.10.06),<br>Full text; particularly, Claim 8; Par. Nos.<br>[0020] to [0021], [0059] to [0060] (text<br>example 1); Par. No. [0061] (test example 2);<br>Par. No. [0063] (preparation example 2);<br>Par. No. [0070] (preparation example 3)<br>(Family: none) | 9-11<br>1-8,12 |

| ☒ | Further documents are listed in the continuation of Box C. | ☐ | See patent family annex. |

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>   26 September, 2007 (26.09.07) | Date of mailing of the international search report<br>   09 October, 2007 (09.10.07) |
|---|---|
| Name and mailing address of the ISA/<br>   Japanese Patent Office | Authorized officer |
| Facsimile No. | Telephone No. |

Form PCT/ISA/210 (second sheet) (April 2005)

21

**INTERNATIONAL SEARCH REPORT**

International application No.

PCT/JP2007/063287

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2001-55343 A (TERUMO CORP.),<br>27 February, 2001 (27.02.01),<br>Full text; particularly, abstract; Claim 5;<br>preparation examples 1 to 14; test examples<br>1 to 11<br>& WO 00/25748 A1     & EP 1044679 A1<br>& US 2003/211142 A1 | 9-11<br>1-8,12 |
| A | JP 2667051 B2 (SEQUUS PHARMACEUTICALS INC.),<br>22 October, 1997 (22.10.97),<br>Full text; particularly, Claim 1<br>(cited in the description of the present<br>application as "patent document 3")<br>& WO 91/05546 A1     & JP 5-501264 A<br>& EP 0496835 A1     & JP 5-505173 A<br>(cited in the description of the present<br>application as "patent document 1")<br>& WO 91/05545 A1     & EP 0496813 A1<br>& US 5225212 A     & AT 115401 T<br>& AT 122229 T     & AT 142483 T<br>& AT 152614 T     & AU 642679 B2<br>& AU 654120 B2     & AU 5323194 A<br>& AU 6527294 A     & AU 6637490 A<br>& AU 6898291 A     & CA 2067133 A1<br>& CA 2067178 A1     & CA 2146565 A1<br>& DE 69015207 D1     & DE 69019366 D1<br>& DE 69304685 D1     & DE 69310527 D1<br>& DK 496835 T3     & DK 632719 T3<br>& EP 0632719 A1     & EP 0662820 A1<br>& ES 2071976 T3     & ES 2092296 T3<br>& ES 2104184 T3     & FI 921764 A<br>& FI 921763 A     & GR 3017060 T3<br>& HK 14097 A     & IL 96069 D0<br>& IL 96070 D0     & JP 10-1431 A<br>& JP 3571335 B2     & JP 2001-181214 A<br>& KR 0134982 B1     & LU 88854 A9<br>& NL 960031 I1     & NO 921214 D0<br>& NO 921213 D0     & US 5013556 A<br>& US 5213804 A     & US 5356633 A<br>& US 5527528 A     & US 5620689 A<br>& US 5843473 A     & US 2001/051183 A1<br>& US 2003/113369 A1     & WO 93/19738 A1<br>& WO 94/07466 A1     & WO 94/22429 A1 | 1-12 |

Form PCT/ISA/210 (continuation of second sheet) (April 2005)

**INTERNATIONAL SEARCH REPORT**

| International application No. |
|---|
| PCT/JP2007/063287 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 7-20857 B (TERUMO CORP.),<br>08 March, 1995 (08.03.95),<br>Full text; particularly, Claim 1<br>(cited in the description of the present<br>application as "patent document 2")<br>& JP 2-149512 A      & EP 0354855 A2<br>& US 5593622 A      & US 5846458 A<br>& US 5846458 A      & AU 616040 B2<br>& AU 640298 B2      & AU 1007792 A<br>& AU 3948789 A      & DE 68919772 D1<br>& ES 2064470 T3 | 1-12 |
| A | JP 10-142214 A (MITSUBISHI CHEMICAL CORP.),<br>29 May, 1998 (29.05.98),<br>Full text; particularly, abstract; Claims<br>1, 4, 6<br>(cited in the description of the present<br>application as "patent document 4")<br>& JP 3778528 B2 | 1-12 |

Form PCT/ISA/210 (continuation of second sheet) (April 2005)

**INTERNATIONAL SEARCH REPORT**

International application No.

PCT/JP2007/063287

---

**Box No. II**      **Observations where certain claims were found unsearchable (Continuation of item 2 of first sheet)**

This international search report has not been established in respect of certain claims under Article 17(2)(a) for the following reasons:

1. ☐   Claims Nos.:
   because they relate to subject matter not required to be searched by this Authority, namely:

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

---

**Box No. III**      **Observations where unity of invention is lacking (Continuation of item 3 of first sheet)**

This International Searching Authority found multiple inventions in this international application, as follows:
     (See extra sheet.)

1. ☐   As all required additional search fees were timely paid by the applicant, this international search report covers all searchable claims.

2. ☒   As all searchable claims could be searched without effort justifying an additional fee, this Authority did not invite payment of any additional fee.

3. ☐   As only some of the required additional search fees were timely paid by the applicant, this international search report covers only those claims for which fees were paid, specifically claims Nos.:

4. ☐   No required additional search fees were timely paid by the applicant. Consequently, this international search report is restricted to the invention first mentioned in the claims; it is covered by claims Nos.:

**Remark on Protest**    ☐   The additional search fees were accompanied by the applicant's protest and, where applicable,
the                       payment of a protest fee..

                           ☐   The additional search fees were accompanied by the applicant's protest but the applicable protest fee was not paid within the time limit specified in the invitation.

                           ☐   No protest accompanied the payment of additional search fees.

Form PCT/ISA/210 (continuation of first sheet (2)) (April 2005)

| **INTERNATIONAL SEARCH REPORT** | International application No. |
|---|---|
| | PCT/JP2007/063287 |

Continuation of Box No.III of continuation of first sheet(2)

This international application is considered to involve the following two inventions.

(1) The subject matters of claims 1-8 and 12
(2) The subject matter of claims 9-11

Claims 1-6 pertain to a method of separating vesicles whose membrane has been modified with a hydrophilic polymer which comprises separating vesicles differing in the degree of membrane modification by ion-exchange chromatography. Claims 7 and 8 pertain to a process for producing vesicles purified so as to have a desired degree of modification with a hydrophilic polymer which comprises utilizing the separation method in the step of purifying vesicles. Claim 12 pertains to a method of evaluating a medicinal vesicle preparation containing a drug therein, the method comprising using the separation method.

In contrast, claims 9-11 pertain to vesicles in which the outer surface of the membrane has been modified with a hydrophilic polymer and which include no vesicle having a degree of hydrophilic-polymer modification less than 0.5 mol% based on the lipid of the vesicle membrane. Such vesicles satisfying the requirement are not limited to the vesicles produced by utilizing the separation method of claims 1-6 in the step of purifying vesicles. In addition, such vesicles are already known as apparent from the fact that they are described in, e.g., JP 2001-55343 A as shown in Box C.

Therefore, there is no technical relationship between the subject matters of claims 1-8 and 12 and the subject matter of claims 9-11 which involves one or more, identical or corresponding special technical features. The claims are hence not considered to be so linked as to form a single general inventive concept.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP HEI5505173 T
- JP HEI720857 B
- JP 2667051 B
- JP HEI10142214 B
- JP SHO61161246 B
- JP HEI5508626 T
- JP HEI2292246 B
- JP HEI4108391 B
- JP 9042166 W

### Non-patent literature cited in the description

- **D.D. LASIC.** LIPOSOMES from Physics to Applications. Elsevier, 1993
- Long Circulating Liposomes: Old Drugs, New Therapeutics. Springer, 1997
- Medical Applcations of LIPOSOMES. Elsevier, 1998
- Effects of mixed polyethylene glycol modification on fixed aqueous layer thickness and antitumor activity of Doxorubicin containing liposome. 2002, vol. 238, 171-180
- Liposome Technology. 1992, vol. I
- Liposomes in Life Science. Springer-Verlag Tokyo, 1992
- **COLIN TILCOCK ; PIETER CULLIS ; TOMAS DEMPSEY.** *B.B.A,* 1989, vol. 979, 208-214
- Liposomes in Life Science. Springer-Verlag Tokyo Inc, 1992